(19) Europäisches Patentamt
European Patent Office
Office européen des brevets

(11) EP 0 717 023 B1

(12) EUROPÄISCHE PATENTSCHRIFT

(45) Veröffentlichungstag und Bekanntmachung des Hinweises auf die Patenterteilung:
**10.02.1999 Patentblatt 1999/06**

(51) Int Cl.⁶: **C07C 29/149**, C07C 31/20, C07C 31/34, C07C 31/22, C07C 31/36

(21) Anmeldenummer: **95118754.1**

(22) Anmeldetag: **29.11.1995**

# (54) Verfahren zur Herstellung von optisch aktiven Alkoholen durch catalytische Reduktion der optisch aktiven Carbonsäuren

Process for the preparation of optically active alcohols through catalytic reduction of optically active carboxylic acids

Procédé de préparation d'alcools optiquement actifs à partir de réduction catalytique d'acides carbocycliques optiquement actifs

(84) Benannte Vertragsstaaten:
**BE CH DE ES FR GB IE IT LI PT**

(30) Priorität: **12.12.1994 DE 4444109**

(43) Veröffentlichungstag der Anmeldung:
**19.06.1996 Patentblatt 1996/25**

(73) Patentinhaber: **BAYER AG**
**51368 Leverkusen (DE)**

(72) Erfinder: **Antons, Stefan, Dr.**
**D-51373 Leverkusen (DE)**

(56) Entgegenhaltungen:
**WO-A-93/19030**
**US-A- 2 607 805**
**US-A- 2 607 807**
**US-A- 4 273 947**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein Verfahren zur Herstellung von optisch aktiven Alkoholen durch Reduktion der entsprechenden optisch aktiven Carbonsäuren.

Es ist bekannt, daß man im Labormaßstab aus optisch reinen Carbonsäuren optisch reine Alkohole herstellen kann, wenn man eine Reduktion mit Lithium-Aluminium-Hydrid oder aktiviertem Natrium-Bor-Hydrid durchführt (siehe Monatshefte der Chemie 83, 541 (1952), Helv. Chim. Acta. 31, 1617 (1949), Chem. Pharm. Bull. 13, 995 (1965), JACS 78, 2582 (1956), JOC 58, 3568 (1993), Angew. Chem. Int. Ed. 28, 218 (1989), Tetrahedron Letters 33, 5517 (1992) und DE-OS 3 827 789). Für eine Anwendung im technischen Maßstab sind die benötigten Reduktionsmittel nicht geeignet, da sie nur mit besonders großem Aufwand gehandhabt werden können und sehr kostenintensiv sind.

Es ist auch bekannt, daß man Carbonsäuren mit Wasserstoff katalytisch zu Alkoholen reduzieren kann. Eine zusammenfassende Darstellung hierzu ist in Houben-Weyl, Methoden der organischen Chemie, 4. Aufl. Bd. VI/1b, Seiten 103-107 (1984) enthalten. Über die Stereoselektivität dieser Reduktion beim Einsatz optisch aktiver Carbonsäuren ist nichts bekannt geworden. Soweit Ruthenium enthaltende Katalysatoren eingesetzt werden (loc. cit. S. 106) sind dabei relativ hohe Temperaturen und sehr hohe Drucke erforderlich z.B. Temperaturen von 145 bis 190°C und Drucke von 700 bis 950 bar. Für die Herstellung optisch aktiver Alkohole sind solche Verfahren nicht geeignet, da bei den anzuwendenden drastischen Reaktionsbedingungen Racemisierungen und Abbaureaktionen stattfinden.

WO-A 93/19 030 beschreibt die Herstellung aus Xylitol aus D-Glucose, D-Fructose, D-Galaktose und Mischungen davon. Über die Stereospezifität dieser Reaktion werden keine Angaben gemacht.

Es besteht deshalb immer noch ein Bedürfnis nach einem gut, einfach und kostengünstig durchzuführenden Verfahren zur Herstellung von optisch aktiven Alkoholen.

Es wurde nun ein Verfahren zur Herstellung von optisch aktiven Alkoholen gefunden, das dadurch gekennzeichnet ist, daß man optisch aktive Carbonsäuren der Formel (I)

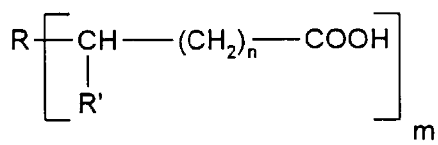

(I),

in der

m für 1, 2 oder 3,

n für null oder eine ganze Zahl von 1 bis 5 und

$R^1$ für einen einbindigen, aus der Gruppe der geradkettigen und verzweigten $C_1$-$C_{12}$-Alkyl-, $C_7$-$C_{12}$-Aralkyl-, $C_6$-$C_{10}$-Aryl- und $C_1$-$C_{12}$-Alkoxyreste ausgewählten Rest oder für Hydroxy oder Halogen stehen und

im Falle m = 1

R für einen einbindigen, aus der Gruppe der geradkettigen und verzweigten $C_1$-$C_{12}$-Alkyl-, $C_7$-$C_{12}$-Aralkyl-, $C_6$-$C_{10}$-Aryl- und $C_1$-$C_{12}$-Alkoxyreste ausgewählten Rest oder für einen Halogen- oder Hydroxyrest steht, der von R' verschieden ist,

im Falle m = 2

R nicht vorhanden ist oder für einen zweibindigen, aus der Gruppe der geradkettigen und verzweigten $C_1$-$C_{12}$-Alkyl- und $C_7$-$C_{12}$-Aralkylreste ausgewählten Rest steht, und

im Falle m = 3

R für einen dreibindigen, aus der Gruppe der geradkettigen und verzweigten $C_1$-$C_{12}$-Alkyl- und $C_7$-$C_{12}$-Aralkylreste ausgewählten Rest steht,

in Gegenwart von Ruthenium-Katalysatoren bei Temperaturen unter 160°C und Drucken unter 250 bar mit Wasserstoff reduziert und optisch aktive Alkohole der Formel (II) erhält

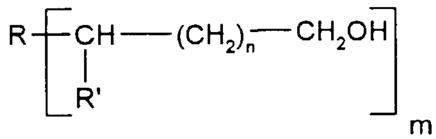

(II),

in der
m, n, R' und R die bei Formel (I) angegebene Bedeutung haben.

In den Formeln (I) und (II) stehen vorzugsweise

| | |
|---|---|
| m | für 1 oder 2, |
| n | für null, 1 oder 2 und |
| $R^1$ | für geradkettiges oder verzweigtes $C_1$-$C_4$-Alkyl, Benzyl, Hydroxy, Fluor, Chlor oder Brom und ist |
| R von R' | verschieden und steht für geradkettiges oder verzweigtes $C_1$-$C_4$-Alkyl, Benzyl, Hydroxy, Fluor oder Chlor. |

Weiterhin ist bevorzugt, daß einer der Reste R' und R für Hydroxy steht.

Wenn m für 2 oder 3 steht, also der Molekülteil $-CH(R^1)-(CH_2)_n-COOH$ im Einsatzmaterial mehrfach vorhanden ist, so können diese Molekülteile hinsichtlich der Bedeutung von n und R' gleich oder verschieden sein.

Soweit R' und R Alkyl-, Aralkyl-, Aryl- und/oder Alkoxyreste sind können diese gegebenenfalls substituiert sein, z.B. mit Halogen-, Hydroxy-, $C_1$-$C_4$-Alkoxy-, Thiol-, Amino- und/oder $C_1$-$C_4$-Alkylaminoresten. Vorzugsweise enthalten R' und/oder R weniger als vier derartige Substituenten. Es ist möglich, daß Reduktionen und/oder Spaltungsreaktionen auch an solchen Substituenten stattfinden.

Soweit R' und R Alkyl-, Aralkyl-, Aryl- oder Alkoxyreste sind können diese in der Alkylkette und/oder im Arylteil gegebenenfalls Heteroatome enthalten, z.B. Sauerstoff-, Schwefel- und/oder Stickstoffatome. Vorzugsweise sind weniger als 3 solcher Heteroatome vorhanden.

Soweit R' und R Aralkyl- oder Arylreste sind können diese auch partiell oder vollständig hydriert sein.

Besonders bevorzugt setzt man in das erfindungsgemäße Verfahren optisch aktive Milchsäure, optisch aktive Weinsäure, optisch aktive 2-Chlorpropionsäure oder optisch aktive Äpfelsäure ein und erhält optisch aktives 1,2-Propandiol, optisch aktives 1,2,3,4-Tetrahydroxybutan, optisch aktives 2-Chlorpropanol bzw. optisch aktives 1,2,4-Trihydroxybutan.

Als Ruthenium-Katalysatoren kommen elementares Ruthenium und Rutheniumverbindungen in Frage, die beide als solche oder aufgebracht auf einem Trägermaterial zum Einsatz gelangen können. Beispiele für Katalysatoren sind fein verteiltes elementares Ruthenium, Rutheniumoxide, Rutheniumhydroxide und Rutheniumhalogenide. Als Trägermaterial kommen beispielsweise Kohlen, Aluminiumoxide, Siliciumdioxide, Silikate, Erdalkalicarbonate und Erdalkalisulfate in Frage. Trägerkatalysatoren können beispielsweise 1 bis 20 Gew.-% elementares Ruthenium oder die entsprechende Menge Rutheniumverbindungen enthalten.

Bezogen auf 1 Mol eingesetzte optisch aktive Carbonsäure kann man z.B. 0,1 bis 10 g elementares Ruthenium oder Rutheniumverbindungen oder 1 bis 50 g Ruthenium enthaltenden Trägerkatalysator einsetzen.

Die erfindungsgemäße Reduktion wird vorzugsweise in Gegenwart eines Lösungsmittels für die optisch aktive Carbonsäure und den optisch aktiven Alkohol durchgeführt. Als Lösungsmittel kommen beispielsweise Wasser, mit Wasser mischbare organische Lösungsmittel und Gemische aus beiden in Frage. Als mit Wasser mischbare Lösungsmittel seien niedere Alkohole und mit Wasser mischbare Ether genannt. Bevorzugte Lösungsmittel sind Wasser und Gemische, die Wasser und niedrige Alkohole oder Tetrahydrofuran enthalten.

Geeignete Reaktionsbedingungen für das erfindungsgemäße Verfahren sind z.B. Temperaturen im Bereich 50 bis 150°C und Drücken im Bereich 5 bis 250 bar. Vorzugsweise arbeitet man bei 70 bis 130°C und 50 bis 220 bar.

Zur Aufarbeitung des Reaktionsgemisches kann man beispielsweise zunächst abkühlen, den Katalysator abtrennen, z.B. durch Filtration, dann die vorhandenen, leicht flüchtigen Bestandteile gegebenenfalls unter schwach vermindertem Druck, abdestillieren und den Rückstand im Vakuum fraktionieren. Den abgetrennten Katalysator kann man wiederverwenden, ebenso das Lösungsmittel.

Das erfindungsgemäße Verfahren kann kontinuierlich oder diskontinuierlich durchgeführt werden.

Die überraschenden Vorteile des erfindungsgemäßen Verfahrens sind, daß damit optisch aktive Alkohole auf einfache Weise, bei relativ niedrigen Temperaturen und Drucken, mit wenig Aufwand und in hoher Selektivität (Enantiomerenüberschuß ee meist über 90 %) zugänglich sind.

**Beispiele**

**Beispiel 1**

In einem 1,3 l Edelstahlautoklaven wurden 4 g Ru-Mohr und 89 g L-(+)-Milchsäure in 700 g Wasser vorgelegt. Nach Spülen mit Stickstoff wurde die Apparatur verschlossen und 100 bar Wasserstoff aufgedrückt. Innerhalb von 2 Stunden wurde auf 80°C erhitzt und der Wasserstoffdruck auf 200 bar erhöht. Bis zum Ende der Wasserstoffaufnahme wurde bei 80°C und 200 bar gerührt, dann auf Raumtemperatur abgekühlt, der Katalysator abfiltriert und das Wasser abdestilliert. Der erhaltene Rückstand wurde unter Stickstoff bei 16 mbar destilliert. Es wurden 64 g L-(+)-1,2-Propandiol (Kp 74°C; $[\alpha]_D^{20}$ + 16,2°; ee >97 %) erhalten.

**Beispiele 2 bis 7**

Es wurde verfahren wie in Beispiel 1, jedoch wurden andere Katalysatoren eingesetzt. Einzelheiten sind aus Tabelle 1 ersichtlich.

Tabelle 1

| Beispiel Nr. | Katalysator | | erhaltenes 1,2-Propandiol | |
|---|---|---|---|---|
| | Art | Menge (g) | Ausbeute (% der Theorie) | ee für L-Form (%) |
| 2 | 10 Gew.-% Ru auf Kohle | 20 | 74 | >97 |
| 2 | bei 150°C reduziertes $RuO_2$ | 2 | 88 | >97 |
| 4 | 5 Gew.-% Ru auf $Al_2O_3$ | 20 | 68 | >97 |
| 5 | bei 150°C reduziertes $RuO_2$ | 10 | 86 | >97 |
| 6 | 5 Gew.-% Ru auf Kohle | 10 | 35 | 97 |
| 7 | 5 Gew.-% Ru auf Kohle | 20 | 64 | >97 |

**Beispiele 8 bis 10**

Es wurde verfahren wie in Beispiel 1, jedoch wurde bei höheren Temperaturen gearbeitet, was zu verbesserten Reaktionszeiten führte.

**Beispiel 8**

110°C, ee des erhaltenen L-1,2-Propandiols 93 %.

**Beispiel 9**

120°C, ee des erhaltenen L-1,2-Propandiols 80 %.

**Beispiel 10**

140°C, ee des erhaltenen L-1,2-Propandiols 71 %.

**Beispiele 11 bis 13**

Es wurde verfahren wie in Beispiel 1, jedoch wurde als Katalysator 5 g Ru-Pulver eingesetzt. Nach jeder Umsetzung wurde der Katalysator zurückgewonnen und erneut in die gleiche Umsetzung eingesetzt. Nach 5-maliger Rückführung wurde der Katalysator nacheinander für Umsetzungen bei 100°C und 200 bar in Gegenwart verschiedener Lösungsmittel eingesetzt.

**Beispiel 11**

700 g eines Gemisches aus 80 Gew.-% Tetrahydrofuran und 20 Gew.-% Wasser; ee des erhaltenen L-1,2-Propandiols 96 %.

**Beispiel 12**

700 g eines Gemisches aus 80 Gew.-% Methanol und 20 Gew.-% Wasser; ee des erhaltenen L-1,2-Propandiols 94 %.

**Beispiel 13**

700 g eines Gemisches aus 80 Gew.-% i-Propanol und 20 Gew.-% Wasser; ee des erhaltenen L-1,2-Propandiols 95 %.

**Beispiel 14**

In einen 1,3 l Edelstahl-Autoklaven wurden 150 g L-Weinsäure, 700 ml Wasser und 20 g Ru-Mohr eingesetzt und bei 200 bar Wasserstoff und 80°C bis zum Ende der Wasserstoffaufnahme gerührt. Nach dem Abkühlen des Reaktionsgemisches, der Entfernung des Katalysators durch Filtration und nach der Entfernung des Wassers durch Destillation wurden 123 g eines klaren Öls erhalten, welches über Nacht bei 4°C auskristallisierte. Die erhaltene kristalline Masse wurde 2 mal aus absolutem Ethanol umkristallisiert. Es wurden 86 g L-1,2,3,4-Butan-tetraol in Form eines reinweißen Feststoffs erhalten (Smp. 87-88°C; $[\alpha]_D^{20}$ + 11,6°; c = 2,EtOH).

**Beispiel 15**

In einem 1,3 l Edelstahl-Autoklaven wurden 135 g L-Äpfelsäure, 700 ml Wasser und 30 g Ru/Mohr eingesetzt und bei 200 bar Wasserstoff und 80°C bis zum Ende der Wasserstoffaufnahme gerührt. Nach Abkühlen, Abtrennung des Katalysators durch Filtration und Abtrennung des Wassers durch Destillation wurden 106 g eines klaren Öles erhalten und dieses wurde bei 1 mbar destilliert und ergab dabei 89 g 97%iges L-1,2,4-Butantriol (Kp 172°C; $[\alpha]_D^{20}$-26,6°; c = 1,MeOH).

**Beispiel 16**

In einem 3 l Edelstahlautoklaven wurden 20 g Ru-Mohr und 109 g S-(-)-2-Chlorpropionsäure in 700 ml Wasser vorgelegt. Nach Spülen mit Stickstoff wurde die Apparatur verschlossen und 100 bar Wasserstoff aufgedrückt. Innerhalb von 2 Stunden wurde auf 140°C erhitzt und der Wasserstoffdruck auf 200 bar erhöht. Bis zum Ende der Wasserstoffaufnahme wurde bei 140°C und 200 bar gerührt, dann auf Raumtemperatur abgekühlt, der Katalysator abfiltriert und das Wasser abdestilliert. Der erhaltene Rückstand wurde unter Stickstoff bei Normaldruck destilliert. Es wurden 72 g S-(+)-2-Chlorpropanol (Kp 160°C; $[\alpha]_D^{20}$ + 14,4°, in Substanz) erhalten mit einer ee = 82,3 %.

**Vergleichsbeispiel**

Es wurde verfahren wie in Beispiel 1, jedoch wurde als Katalysator Kupfer-Chromit eingesetzt (ein für Reduktionen von Carbonsäuren zu Alkoholen üblicher Hydrierkatalysator). Bis 150°C fand keine Umsetzung statt, oberhalb 160°C traten neben 1,2-Propandiol weitere Reaktionsprodukte auf und das 1,2-Propandiol war weitgehend racemisiert.

In einem weiteren Ansatz wurde als Katalysator Raney-Nickel eingesetzt (ein üblicher Katalysator für die Reduktion von Carbonsäureestern zu Alkoholen) Während der Umsetzung löste sich das Nickel teilweise auf und aus dem Reaktionsgemisch konnte keine 1,2-Propandiol isoliert werden.

**Patentansprüche**

1. Verfahren zur Herstellung von optisch aktiven Alkoholen, dadurch gekennzeichnet, daß man optisch aktive Carbonsäuren der Formel (I)

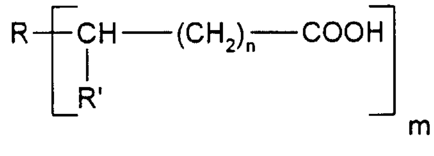

(I),

in der

m für 1, 2 oder 3,

n für null oder eine ganze Zahl von 1 bis 5 und

$R^1$ für einen einbindigen, aus der Gruppe der geradkettigen und verzweigten $C_1$-$C_{12}$-Alkyl, $C_7$-$C_{12}$-Aralkyl-, $C_6$-$C_{10}$-Aryl- und $C_1$-$C_{12}$-Alkoxyreste ausgewählten Rest oder für Hydroxy oder Halogen stehen und

im Falle m = 1

R für einen einbindigen, aus der Gruppe der geradkettigen und verzweigten $C_1$-$C_{12}$-Alkyl-, $C_7$-$C_{12}$-Aralkyl-, $C_6$-$C_{10}$-Aryl- und $C_1$-$C_{12}$-Alkoxyreste ausgewählten Rest oder für einen Halogen- oder Hydroxyrest steht, der von R' verschieden ist,

im Falle m = 2

R nicht vorhanden ist oder für einen zweibindigen, aus der Gruppe der geradkettigen und verzweigten $C_1$-$C_{12}$-Alkyl- und $C_7$-$C_{12}$-Aralkylreste ausgewählten Rest steht, und

im Falle m = 3

R für einen dreibindigen, aus der Gruppe der geradkettigen und verzweigten $C_1$-$C_{12}$-Alkyl- und $C_7$-$C_{12}$-Aralkylreste ausgewählten Rest steht,

in Gegenwart von Ruthenium-Katalysatoren bei Temperaturen unter 160°C und Drucken unter 250 bar mit Wasserstoff reduziert und optisch aktive Alkohole der Formel (II) erhält

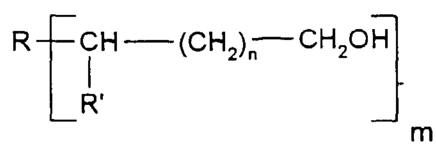

(II),

in der
m, n, R' und R die bei Formel (I) angegebene Bedeutung haben.

**2.** Verfahren nach Anspruch 1, dadurch gekennzeichnet, daß in den Formeln (I) und (II)

m für 1 oder 2,

n für null, 1 oder 2 und

R' für geradkettiges oder verzweigtes $C_1$-$C_4$-Alkyl, Benzyl oder einen Hydroxy, Fluor, Chlor oder Brom stehen und

R von R' verschieden ist und für geradkettiges oder verzweigtes $C_1$-$C_4$-Alkyl, Benzyl, Hydroxyl, Fluor oder Chlor steht.

**3.** Verfahren nach Ansprüchen 1 bis 2, dadurch gekennzeichnet, daß einer der Reste R' und R für Hydroxy steht.

**4.** Verfahren nach Ansprüchen 1 bis 3, dadurch gekennzeichnet, daß R' und R, soweit sie für Alkyl-, Aralkyl-, Aryl- und/oder Alkoxyreste stehen mit Halogen-, Hydroxy-, $C_1$-$C_4$-Alkoxy-, Thiol-, Amino- und/oder $C_1$-$C_4$-Alkylaminoresten substituiert sind und/oder in der Alkylkette und/oder im Arylteil Heteroatome enthalten.

**5.** Verfahren nach Ansprüchen 1 bis 4, dadurch gekennzeichnet, daß man als optisch aktive Carbonsäure, optisch aktive Milchsäure, optisch aktive Weinsäure, optisch aktive 2-Chlorpropionsäure oder optisch aktive Äpfelsäure

einsetzt und optisch aktives 1,2-Propandiol, optisch aktives 1,2,3,4-Tetrahydroxybutan, optisch aktives 2-Chlorpropanol bzw. optisch aktives 1,2,4-Trihydroxybutan erhält.

**6.** Verfahren nach Ansprüchen 1 bis 5, dadurch gekennzeichnet, daß man als Rutheniumkatalysatoren elementares Ruthenium oder Ruthenium-Verbindungen als solche oder aufgebracht auf einem Trägermaterial einsetzt.

**7.** Verfahren nach Ansprüchen 1 bis 6, dadurch gekennzeichnet, daß man bezogen auf 1 Mol optisch aktive Carbonsäure 0,1 bis 10 g elementares Ruthenium oder Ruthenium-Verbindungen oder 1 bis 50 Ruthenium enthaltende Trägerkatalysatoren einsetzt.

**8.** Verfahren nach Ansprüchen 1 bis 7, dadurch gekennzeichnet, daß man in Gegenwart von Wasser, mit Wasser mischbaren Lösungsmitteln oder Gemischen aus beiden arbeitet.

**9.** Verfahren nach Ansprüchen 1 bis 8, dadurch gekennzeichnet, daß man bei Temperaturen im Bereich 50 bis 150°C und bei Drucken im Bereich 5 bis 250 bar arbeitet.

**10.** Verfahren nach Ansprüchen 1 bis 9, dadurch gekennzeichnet, daß man das Reaktionsgemisch aufarbeitet, indem man zunächst abkühlt, den Katalysator abtrennt, die vorhandenen leicht flüchtigen Bestandteile durch Destillation, gegebenenfalls unter schwach vermindertem Druck abtrennt und den Rückstand im Vakuum fraktioniert.

**Claims**

**1.** Process for the preparation of optically active alcohols, characterized in that optically active carboxylic acids of the formula (I)

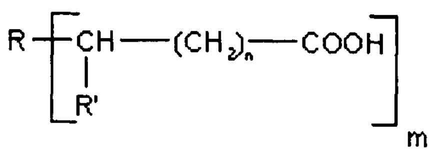

(I),

in which

m is 1, 2 or 3,

n is zero or an integer from 1 to 5 and

R' is a monovalent radical selected from the group comprising linear and branched $C_1$-$C_{12}$-alkyl, $C_7$-$C_{12}$-aralkyl, $C_6$-$C_{10}$-aryl and $C_1$-$C_{12}$-alkoxy radicals or is hydroxyl or halogen, and

if m = 1

R is a monovalent radical selected from the group comprising linear and branched $C_1$-$C_{12}$-alkyl, $C_7$-$C_{12}$-aralkyl, $C_6$-$C_{10}$-aryl and $C_1$-$C_{12}$-alkoxy radicals or is a halogen or hydroxyl radical which is different from R',

if m=2

R is absent or is a divalent radical selected from the group comprising linear and branched $C_1$-$C_{12}$-alkyl and $C_7$-$C_{12}$-aralkyl radicals, and

if m=3

R is a trivalent radical selected from the group comprising linear and branched $C_1$-$C_{12}$-alkyl and $C_7$-$C_{12}$-aralkyl radicals,
are reduced with hydrogen at temperatures below 160°C and pressures below 250 bar in the presence of

ruthenium catalysts, giving optically active alcohols of the formula (II)

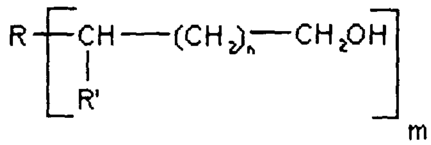
(II),

in which
m, n, R' and R are as defined for the formula (I).

**2.** Process according to Claim 1, characterized in that, in the formulae (I) and (II),

m is 1 or 2,

n is zero, 1 or 2 and

R' is linear or branched $C_1$-$C_4$-alkyl, benzyl or a hydroxyl, fluorine, chlorine or bromine, and

R is different from R' and is linear or branched $C_1$-$C_4$-alkyl, benzyl, hydroxyl, fluorine or chlorine.

**3.** Process according to Claims 1 or 2, characterized in that one of the radicals R' and R is hydroxyl.

**4.** Process according to Claims 1 to 3, characterized in that if R' and R are alkyl, aralkyl, aryl and/or alkoxy radicals, they are substituted by halogen, hydroxyl, $C_1$-$C_4$-alkoxy, thiol, amino and/or $C_1$-$C_4$-alkylamino radicals and/or contain heteroatoms in the alkyl chain and/or in the aryl moiety.

**5.** Process according to Claims 1 to 4, characterized in that the optically active carboxylic acid used is optically active lactic acid, optically active tartaric acid, optically active 2-chloropropionic acid or optically active malic acid, giving optically active propane-1,2-dial, optically active 1,2,3,4-tetrahydroxybutane, optically active 2-chloropropanol or optically active 1,2,4-trihydroxybutane respectively.

**6.** Process according to Claims 1 to 5, characterized in that the ruthenium catalyst used is elemental ruthenium or ruthenium compounds, as such or applied to a support.

**7.** Process according to Claims 1 to 6, characterized in that, based on 1 mol of optically active carboxylic acid, 0.1 to 10 g of elemental ruthenium or ruthenium compounds or 1 to 50 g of ruthenium-containing supported catalysts are used.

**8.** Process according to Claims 1 to 7, characterized in that it is carried out in the presence of water, water-miscible solvents or mixtures of the two.

**9.** Process according to Claims 1 to 8, characterized in that it is carried out at temperatures in the range 50 to 150EC and at pressures in the range 5 to 250 bar.

**10.** Process according to Claims 1 to 9, characterized in that the reaction mixture is worked up firstly by cooling and separation of the catalyst, separation of the readily volatile constituents present by distillation, if necessary under slightly reduced pressure, and fractionation of the residue under vacuum.

**Revendications**

**1.** Procédé pour la préparation d'alcools à l'état d'isomères optiques, caractérisé en ce que l'on réduit des acides carboxyliques à l'état d'isomères optiques, de formule (I)

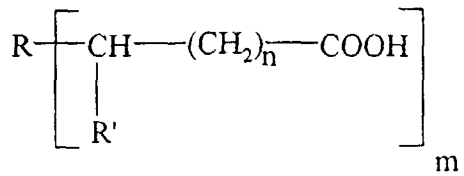 (I),

dans laquelle

m est égal à 1, 2 ou 3,
n est égal à 0 ou représente un nombre entier allant de 1 à 5 et
R' représente un radical à une liaison choisi parmi les radicaux alkyle à chaîne droite ou ramifiée en $C_1$-$C_{12}$, aralkyle en $C_7$-$C_{12}$, aryle en $C_6$-$C_{10}$ et alcoxy en $C_1$-$C_{12}$ ou bien un groupe hydroxy ou un halogène et

dans le cas où m = 1

R représente un radical à une liaison choisie parmi les radicaux alkyle à chaîne droite ou ramifiée en $C_1$-$C_{12}$, aralkyle en $C_7$-$C_{12}$, aryle en $C_6$-$C_{10}$ et alcoxy en $C_1$-$C_{12}$ ou bien un halogène ou un groupe hydroxy, sa signification étant différente de celle de R',

dans le cas où m = 2

R n'existe pas ou représente un radical à deux liaisons, choisi parmi les radicaux alkyle à chaîne droite ou ramifiée en $C_1$-$C_{12}$, et aralkyle en $C_7$-$C_{12}$, et

dans le cas où m = 3

R représente un radical à trois liaisons, choisi parmi les radicaux alkyle à chaîne droite ou ramifiée en $C_1$-$C_{12}$ et aralkyle en $C_7$-$C_{12}$,

par l'hydrogène en présence de catalyseurs au ruthénium à des températures inférieures à 160°C et des pressions inférieures à 250 bars, ce qui donne des alcools à l'état d'isomères optiques de formule (II)

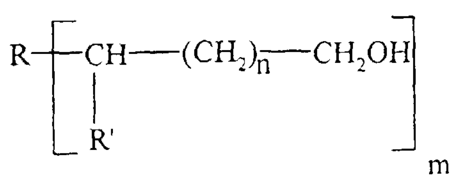 (II),

dans laquelle
m, n, R' et R ont les significations indiquées en référence à la formule (I).

**2.** Procédé selon revendication 1, caractérisé en ce que, dans les formules (I) et (II),

| | |
|---|---|
| m | est égal à 1 ou 2, |
| n | est égal à 0, 1 ou 2 et |
| R' | représente un groupe alkyle à chaîne droite ou ramifiée en $C_1$-$C_4$, benzyle ou un groupe hydroxy, le fluor, le chlore ou le brome et |
| R, différent de R', | représente un groupe alkyle à chaîne droite ou ramifiée en $C_1$-$C_4$, benzyle, un groupe hydroxy, le fluor ou le chlore. |

**3.** Procédé selon les revendications 1 à 2, caractérisé en ce que l'un des symboles R' et R représente un groupe hydroxy.

**4.** Procédé selon les revendication 1 à 3, caractérisé en ce que R' et R, lorsqu'ils représentent des groupes alkyle,

aralkyle, aryle ou alcoxy, peuvent être substitués par des halogènes, des groupes hydroxy, alcoxy en $C_1$-$C_4$, thiol, amino et/ou alkylamino en $C_1$-$C_4$ et/ou contenir des hétéroatomes dans la chaîne alkyle ou dans la partie aryle.

**5.** Procédé selon les revendications 1 à 4, caractérisé en ce que l'acide carboxylique à l'état d'isomère optique mis en oeuvre est de l'acide lactique à l'état d'isomère optique, de l'acide tartrique à l'état d'isomère optique, de l'acide 2-chloropropionique à l'état d'isomère optique ou de l'acide malique à l'état d'isomère optique, et on obtient respectivement le 1,2-propanediol à l'état d'isomère optique, le 1,2,3,4-tétrahydroxybutane à l'état d'isomère optique, le 2-chloropropanol à l'état d'isomère optique et le 1,2,4-trihydroxybutane à l'état d'isomère optique.

**6.** Procédé selon les revendications 1 à 5, caractérisé en ce que le catalyseur au ruthénium consiste en ruthénium élémentaire ou en un dérivé du ruthénium, tels quels ou appliqués sur une matière de support.

**7.** Procédé selon les revendications 1 à 6, caractérisé en ce que, pour 1 mol d'acide carboxylique à l'état d'isomère optique, on utilise de 0,1 à 10 g de ruthénium élémentaire ou de dérivé de ruthénium ou 1 à 50 g d'un catalyseur sur support contenant du ruthénium.

**8.** Procédé selon les revendications l à 7, caractérisé en ce que l'on opère en présence d'eau, de solvants miscibles à l'eau, ou leurs mélanges.

**9.** Procédé selon les revendications 1 à 8, caractérisé en ce que l'on opère à des températures de 50 à 150°C et des pressions de 5 à 250 bar.

**10.** Procédé selon les revendications 1 à 9, caractérisé en ce que, pour l'isolement du produit recherché à partir du mélange de réaction, on commence par refroidir, on sépare le catalyseur, on élimine les constituants volatils par distillation, éventuellement sous vide, et on fractionne le résidu sous vide.